(19) Europäisches Patentamt
European Patent Office
Office européen des brevets

(11) Numéro de publication: **0 017 525**
**B1**

(12) # FASCICULE DE BREVET EUROPEEN

(45) Date de publication du fascicule du brevet: **28.09.83**

(21) Numéro de dépôt: **80400330.9**

(22) Date de dépôt: **13.03.80**

(51) Int. Cl.³: **A 61 K 31/33,**
C 07 D 491/22
//(C07D491/22, 311/00,
221/00, 221/00, 209/00)

(54) **Nouveaux dérivés tétrahydroalstoniques, leur préparation et leur application en tant que médicaments.**

(30) Priorité: **14.03.79 FR 7906469**

(43) Date de publication de la demande:
**15.10.80 Bulletin 80/21**

(45) Mention de la délivrance du brevet:
**28.09.83 Bulletin 83/39**

(84) Etats contractants désignés:
**AT BE CH DE GB IT LU NL SE**

(56) Documents cités:
**FR - A - 2 422 664**

**CHEMICAL ABSTRACTS, vol. 90 (1979) ref. 23358v, page 684 Columbus, Ohio, US S. SAKAI et al.: "The partial synthesis of heteroyohimbine alkaloids; akuammigine, 3-isorauniticine and corynanthe alkaloid; corynantheidine"**

**Le dossier contient des informations techniques présentées postérieurement au dépôt de la demande et ne figurant pas dans le présent fascicule.**

(73) Titulaire: **PIERRE FABRE S.A.**
**125, rue de la Faisanderie**
**F-75116 Paris (FR)**

(72) Inventeur: **Hatinguais, Philippe**
**Le Landou Chemin de Bel Air**
**F-81100 Castres (FR)**
Inventeur: **Cousse, Henri**
**La Foun de los Nobios Chemin de Lastinos**
**F-81100 Castres (FR)**
Inventeur: **Vilain, Pol**
**Les Gaux**
**F-81290 Labruguiere (FR)**

(74) Mandataire: **Corre, Jacques Denis Paul et al,**
**Cabinet Regimbeau 26, Avenue Kléber**
**F-75116 Paris (FR)**

**EP 0 017 525 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Nouveaux dérivés tétrahydroalstoniques, leur préparation et leur application en tant que médicaments

La présente invention, réalisée au Centre de Recherches PIERRE FABRE, concerne de nouveaux dérivés tétrahydroalstoniques répondant à la formule générale I

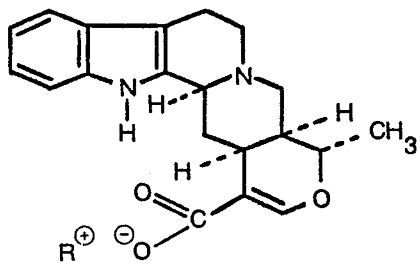

(I)

dans laquelle:

$R^{\oplus}$ représente un ion alcalin ou alcalino-terreux, l'ion ammonium ou bien l'un des ions suivants:

a)

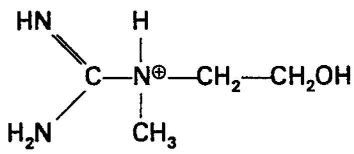

b)

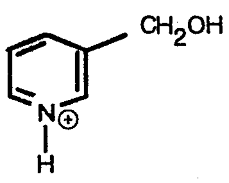

c) $H_3N^{\oplus}—CH_2—CH_2OH$

d)

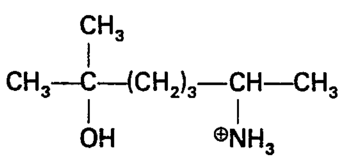

e)

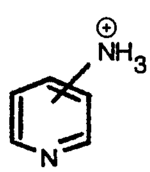

f)

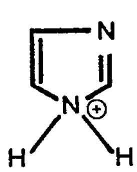

La présente invention concerne également la préparation des composés de formule générale I conformément au schéma réactionnel suivant:

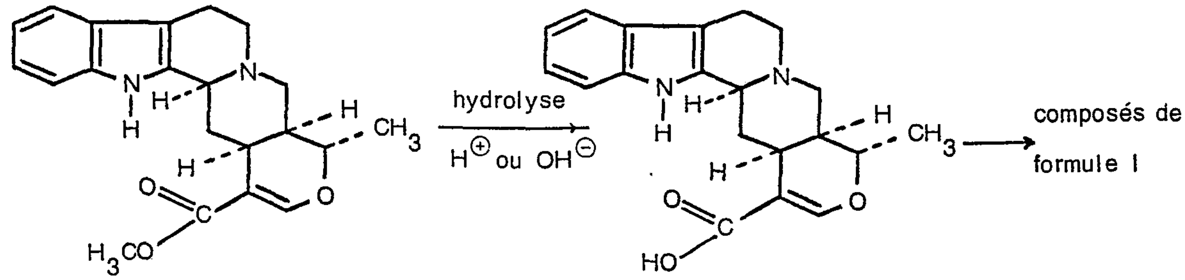


tétrahydroalstonine — tétrahydroalstonique

La tétrahydroalstonine de départ peut par exemple être avantageusement préparée conformément au procédé decrit dans FR—A—2.397.415.

Conformément au procédé de l'invention on réalise une hydrolyse, en milieu acide ou en milieu basique, de la tétrahydroalstonine de formula II

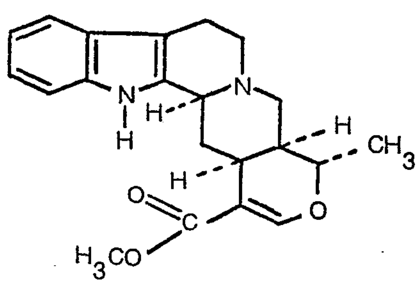

(II)

et, le cas échéant, on neutralise le sel ainsi obtenu pour conduire à l'acide tétrahydroalstonique, qui, le cas echéant, est salifié au moyen d'une base organique ou minérale appropriée.

Selon une caractéristique du procédé de la présente invention, on saponifie la tétrahydroalstonine de formule II, par exemple au moyen de soude, pour conduire au tétrahydroalstonate correspondant, c'est-à-dire par exemple au tétrahydroalstonate de sodium. Ce dernier composé peut alors être trans-salifié par exemple au moyen du chlorhydrate d'une base organique appropriée. La trans-salification est avantageusement réalisée en milieu non aqueux.

Lorsque l'on réalise l'hydrolyse acide de la tétrahydroalstonine de formule II on obtient, par exemple en présence d'acide chlorhydrique, le chlorhydrate correspondant qu'il faut alors neutraliser pour obtenir l'acide tétrahydroalstonique, lequel peut alors être salifié comme précédemment, par traitement avec une quantité stoechiométrique d'une base minérale ou organique appropriée.

Les dérivés tétrahydroalstoniques de formule I se sont révélés posséder une activité pharmacologique fort intéressante. Ils présentent en particulier une activité adrénolytique qui permet de les utiliser en thérapeutique humaine et/ou vétérinaire et, plus particulièrement, dans le traitement des insuffisances circulatoires cérébrales, des hypertensions et de ses manifestations fonctionnelles telles que les vertiges, céphalées, bourdonnements d'oreilles, etc.

La présente invention sera décrite ci-après plus en détail, à propos des quelques exemples particulieurs suivants indiqués à simple titre d'illustration.

Exemple 1

Préparation de l'acide tétrahydroalstonique

*1) Hydrolyse en milieu basique:*

50 g de tétrahydroalstonine sont mis en suspension dans 1500 ml de butanol aqueux contenant 25 g de soude. Le solvant est porté à l'ébullition pendant environ 5 heures. Après addition de 2 litres d'eau, on élimine l'azétrope sous pression réduite. La phase aqueuse résiduelle est amenée à 4 litres avec de l'eau.

On ajuste le pH à 8 avec de l'acide acétique et on ajoute 4 litres de chlorure de méthylène. La solution est amenée à pH 6—7 par une seconde addition d'acide acétique. Le chlorure de méthylène est décanté et une deuxième extraction est réalisée avec 2 litres de solvant. Les phases organiques groupées sont réunies séchées sur $Na_2SO_4$ et concentrées à 500 ml. L'acide tétrahydroalstonique est alors précipité par addition d'hexane. Rendement: 85 à 90%.

*2) hydrolyse acide:*

5 g de tétrahydroalstonine sont mis en suspension dans 500 ml d'acide chlorhydrique 2N. Le mélange est porté à reflux pendant 6 heures. Après refroidissement la solution d'hydrolyse est amenée

à pH 9 avec de la soude et extraite au chlorure de méthylène afin d'éliminer la tétrahydroalstonine non hydrolysée.

Après neutralisation à pH 6—6,5 avec de l'acide acétique la phase aqueuse est extraite, trois fois, par 300 ml de chlorure de méthylène. Les phases organiques réunies sont séchées sur $Na_2SO_4$, concentrées à 50 ml et l'acide tétrahydroalstonique est précipité par addition d'hexane.

L'acide tétrahydroalstonique obtenu répond à la formule développée suivante:

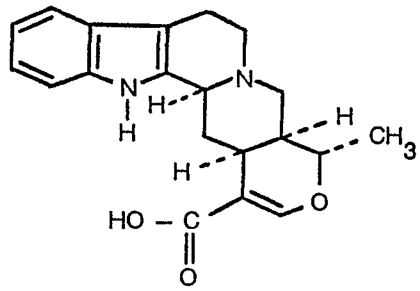

Masse: 338,42
Formule brute: $C_{20}H_{22}N_2O_3$
Point de fusion: 206°C (avec décomposition)
Chromatographie sur plaque:
support: gel de silice
solvant: méthanol-éther éthylique-hexane (20—80—120)
révélateur: UV et/ou vapeurs d'iode
Rf: 0,30
Rx/THA: 0,57
Spectrographie infra rouge à 1% dans KBr: principales bandes d'absorption à 1400— 1450— 1520 et 1630 $cm^{-1}$.

Exemple 2

Tétrahydroalstonate de sodium

Comme indiqué précédemment, le tétrahydroalstonate de sodium peut avantageusement être obtenu directement en hydrolysant la tétrahydroalstonine en présence de soude. Le composé obtenu présente des propriétés physico-chimiques suivantes:
Formule brute: $C_{20}H_{21}N_2O_3Na$
Point de fusion: 250°C (avec décomposition)
Solubilités: eau, 9%
éthanol à 95: 7%
chloroforme: 3,5%
Chromatographie sur plaque:
support: gel de silice
solvant: $CHCl_3$-méthanol-eau (65—25—4)
révélation: UV (254 nm)
Rf: 0,90 (identique à l'acide)
Spectrographie infra rouge à 1% dans KBr: principales bandes d'absorption à 1400 — 1450— 1520— 1630 $cm^{-1}$.

Exemple 3

Tétrahydroalstonate de l'alcool de formule $HN{=}C(NH_2){-}N(CH_3)CH_2{-}CH_2OH$, correspondant à la créatine

50 g de tétrahydroalstonine sont mis en suspension dans 1500 ml de butanol aqueux contenant 25 g de soude et saponifiés comme décrit précédemment. Les jus d'extraction sont groupés, séchés sur $Na_2SO_4$ et concentrés sur 2 litres d'acétone.

D'autre part 23,5 g de sulfate de l'alcool correspondant à la créatine sont solubilisés dans 25 ml d'eau et traités par la quantité stoechiométrique de soude (soit 5,6 g). On concentre à sec sous vide et l'on reprend par 500 ml d'acétone. On filtre le sulfate de sodium et on ajoute la solution d'alcool correspondant à la créatine à la solution d'acide tétrahydroalstonique obtenue précédemment.

On concentre à 500 ml sous vide et on ajoute 200 ml d'hexane. On laisse précipiter une nuit à 5°C et on filtre le précipité. On lave avec un mélange acétone-hexane (6—4) et on obtient un rendement en tétrahydroalstonate d'alcool correspondant à la créatine de 70 à 75%. Le composé obtenu répond à la formule suivante:

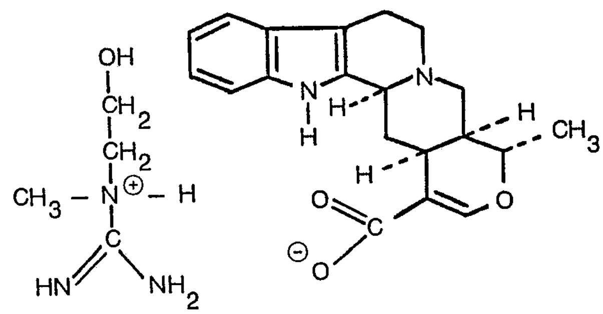

et présente les propriétés physico-chimiques suivantes:

Formule brute: $C_{24}H_{33}N_5O_4$

Point de fusion: 225°C (avec décomposition)

Solubilités: eau 5%

éthanol à 95°: 5%

Chromatographie sur plaque:

support: gel de silice

solvant: $CHCl_3$-méthanol-eau (65—25—4)

Rf: 0,90 (acide)

0,15 (alcool correspondant à la créatine)

Spectrographie infra rouge à 1% dans KBr: principales bandes d'absorption à 1400—1450—1520—1630 $cm^{-1}$.

Spectre RMN:

— méthyle secondaire (porté par le carbone 19) à 1,28 ppm (doublet: J=7Hz)

— méthyle (N—$CH_3$) 2,95 ppm (singélet)

— 4 protons (protons méthyléniques de l'alcool correspondant à la créatine) centrés à 3,55 ppm (multiplet)

— 4 protons aromatiques entre 6,9 et 7,5 ppm.

## Exemple 4

Tétrahydroalstonate d'imidazole

En opérant comme à l'exemple 3, en présence de la base organique correspondante on obtient le tétrahydroalstonate d'imidazole répondant à la formule

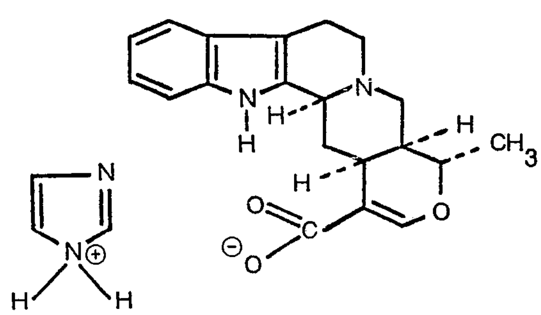

Le composé obtenu présente les propriétés physico-chimiques suivantes:

Formule brute: $C_{23}H_{26}N_4O_3$

Chromatographie sur plaque:

support: gel de silice

solvant: $CHCl_3$-méthanol-eau (65—25—4)

Rf: 0,90 (acide)

0,80 (imidazole)

Spectrographie infra rouge: le spectre infra rouge à 1% dans KBr comporte les principales bandes d'absorption suivantes: 1400— 1450— 1520— 1630 $cm^{-1}$.

## Exemples 5 à 7

En opérant comme précédemment indiqué à l'exemple 3, en présence de la base organique appropriée, on obtient le tétrahydroalstonate d'heptaminol ou amino 6-méthyl 2-heptanol 2, le tétrahydroalstonate d'amino-éthanol ainsi que le tétrahydroalstonate d'amino-pyridine.

Propriétés pharmacologiques:

Les composés de formule I selon la présente invention ont fait l'objet d'expérimentations en vue de déterminer leurs propriétés pharmacologiques et les possibilités d'utilisation en thérapeutique humaine et/ou vétérinaire.

*A) Toxicologie:*

La toxicité aiguë a été recherchée chez la souris par voie orale, et la dose létale 50 calculée par la méthode de Miller et Tainter s'est révélée supérieure à 500 mg/kg par voie orale pour tous les dérivés de formule I testés.

Par voie injectable les composés de formule I ont été administrés jusqu'à 100 mg/kg, sans provoquer de mortalité chez le rat. A titre d'exemple, on précisera que la DL 50 par voie intraveineuse chez la souris est de 330 mg/kg pour le tétrahydroalstonate de sodium. La toxicité du tétrahydroalstonate de l'alcool correspondant à la créatine chez la souris, par voie intraveineuse calculée selon la méthode de Kärber est de 450 mg/kg.

*B) Etude pharmacodynamique:*

a) activité adrénolytique

Les propriétés adrénolytiques ont été recherchées chez le chat et la chien anesthésiés vis-à-vis de la pression artérielle et de la membrane nictitante (chat).

1) résultats chez le chat:

Après administration unique, tous les dérivés de formule générale I présentent une activité à la dose de 5 mg/kg par voie intraveineuse.

Les composés de formule I les plus actifs sont le tétrahydroalstonate de sodium et le tétrahydroalstonate de l'alcool correspondant à la créatine qui agissent par voie injectable à la dose de 0,1 mg/kg. Cette activité est très nettement supérieure à celle de la raubasine et de la tétrahydroalstonine; les composés les plus actifs de formule I sont de 10 à 20 fois plus actifs que la raubasine.

L'adrénolyse est en effet parfaitement nette pour les doses suivantes:

— tétrahydroalstonate de l'alcool correspondant à la créatine: 50 $\mu$g à 100 $\mu$g/kg
— raubasine: 2 mg/kg
— tétrahydroalstonine: 0,5 mg/kg.

2) Etude chez le chien:

Le tétrahydroalstonate de sodium et le tétrahydroalstonate de l'alcool correspondant à la créatine entraînent une diminution progressive de la pression artérielle proportionnelle à la dose de 0,1 à 10 mg/kg administrée par voie intraveineuse.

Cette activité ne s'accompagne pas de diminution de la force de contraction cardiaque. L'inversion de l'hypertension à l'adrénaline commence dès la dose de 0,05 mg/kg.

3) Etude par voie orale chez le rat:

A la dose de 100 mg par voie orale chez le rat le tétrahydroalstonate de sodium et le tétrahydroalstonate de l'alcool correspondant à la créatine réduisent l'augmentation de l'excrétion urinaire provoquée par l'adrénaline. Cette action confirme les propriétés adrénolytiques et démontre également la bonne absorption par voie orale.

b) pharmacologie générale:

Ces dérivés de formule générale I sont dépourvus d'actions vis-à-vis des paramètres biochimiques glycémie, cholestérol.

Les tétrahydroalstonates de sodium, de l'alcool correspondant à la créatine et d'imidazole sont actifs in vitro vis-à-vis de l'agrégation à l'ADP, au collagène et à l'ADP + adrénaline; les concentrations actives variant de $1.10^{-3}$M à $1.10^{-5}$M selon les tests et les composés particuliers répondant à la formule générale I.

On remarquera par ailleurs que ces composés sont en outre faiblement ou pas du tout diurétiques.

Utilisation thérapeutique:

Ces dérivés de formule générale I et plus particulièrement les tétrahydroalstonates de l'alcool correspondant à la créatine, d'imidazole et de sodium peuvent être utilisés dans le traitement des insuffisances circulatoires cérébrales et/ou pour le traitement des hypertensions.

Composition pharmaceutique:

A titre de simple illustration figurent ci-après quelques exemples de formulation de médicaments renfermant au moins un principe actif choisi les composées de formule générale I. Ces principes actifs peuvent être utilisés seuls ou en association avec d'autres principes actifs thérapeutiquement acceptables qui peuvent compléter éventuellement leur spectre d'activité.

Exemple A

Comprimés de tétrahydroalstonate de sodium dosés à 25, 50 et 100 mg.

Exemple B

Flacons de 50 ml contenant 1 g de tétrahydroalstonate de l'alcool correspondant à la créatine pour administration orale sous forme de gouttes.

Exemple C

Ampoules injectables dosées à 5 et 10 mg.

Exemple D

Comprimés de tétrahydroalstonate d'imidazole comprenant:
— 2 à 50 mg de tétrahydroalstonate d'imidazole, et
— 25 mg de dipyridamole.

Exemple E

Flacons de 50 ml renfermant:
— 1000 mg de tétrahydroalstonate de l'alcool correspondant à la créatine, et
— de lavitamine B15 et/ou de la vitamine B6, à raison de 50 à 100 mg.

Exemple F

Comprimés renfermant:
— 2 à 50 mg de tétrahydroalstonate de sodium, et
— 20 mg d'un agent bêta-bloquant, tel que le propranolol.

**Revendications pour les Etats contractants: BE CH DE GB IT LU NL SE**

1. Les composés répondant à la formule générale I:

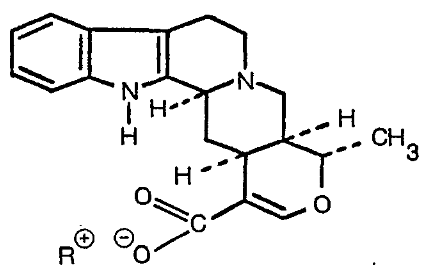

(I)

dans laquelle:

$R^{\oplus}$ représente un ion alcalin ou alcalino-terreux, l'ion ammonium ou bien l'un des ions suivants:

a)

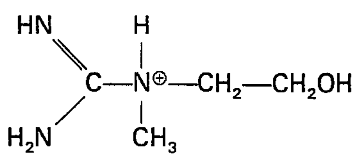

b)

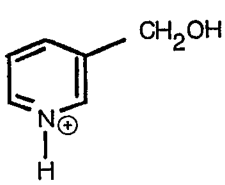

c) $H_3N^{\oplus}—CH_2—CH_2OH$

d)

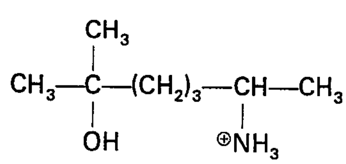

e) 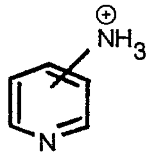

f) 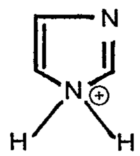

2. Procédé de préparation des composés de formule générale I telle que définie à la revendication 1, caractérisé par une hydrolyse basique de la tétrahydroalstonine de formule II

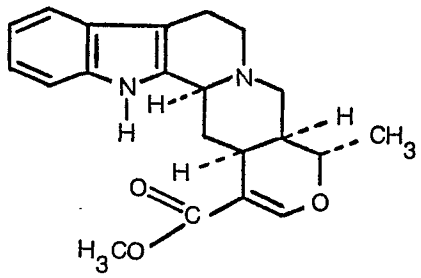

(II)

et le cas échéant une acidification conduisant à l'acide tétrahydroalstonique qui est ensuite salifié au moyen d'une base organique ou minérale appropriée.

3. Procédé selon la revendication 2, caractérisé en ce qu'on saponifie la tétrahydroalstonine au moyen de soude pour obtenir le tétrahydroalstonate de sodium.

4. Procédé selon la revendication 3, caractérisé en ce que le tétrahydroalstonate de sodium est trans-salifié.

5. Procédé selon la revendication 4, caractérisé en ce qu la trans-salification est mise en oeuvre par le chlorhydrate de la base organique appropriée.

6. A titre de médicaments nouveaux les composés de formule générale I selon la revendication 1.

**Revendications pour l'Etat contractant: AT**

1. Procédé de préparation des composés répondant à la formule générale I:

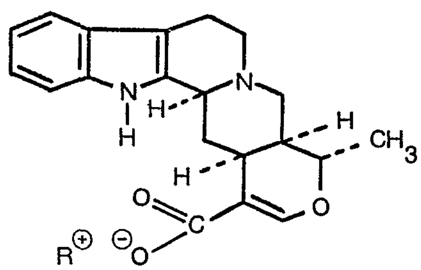

(I)

dans laquelle:

R⊕ représente un ion alcalin ou alcalino-terreux, l'ion ammonium ou bien l'un des ions suivants:

a)

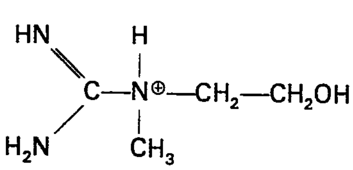

b) 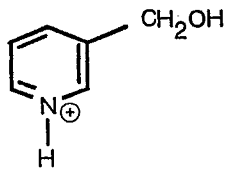

c) $H_3N^{\oplus}—CH_2—CH_2OH$

d) 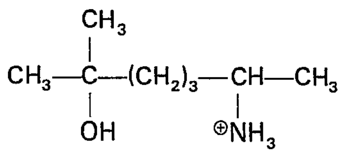

e) 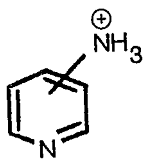

f) 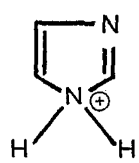

caractérisé par une hydrolyse basique de la tétrahydroalstonine de formule II

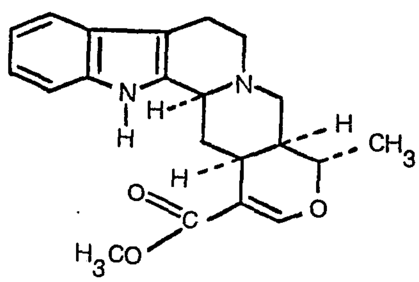

(II)

et le cas échéant une acidification conduisant à l'acide tétrahydroalstonique qui est ensuite salifié au moyen d'une base organique ou minérale appropriée.

2. Procédé selon la revendication 1, caractérisé en ce qu'on saponifie la tétrahydroalstonine au moyen de soude pour obtenir le tétrahydroalstonate de sodium.

3. Procédé selon la revendication 2, caractérisé en ce que le tétrahydroalstonate de sodium est trans-salifié.

4. Procédé selon la revendication 3, caractérisé en ce que la trans-salification est mise en oeuvre par le chlorhydrate de la base organique appropriée.

**Claims for the Contracting States: BE CH DE GB IT LU NL SE**

1. Compounds corresponding to the general formula I:

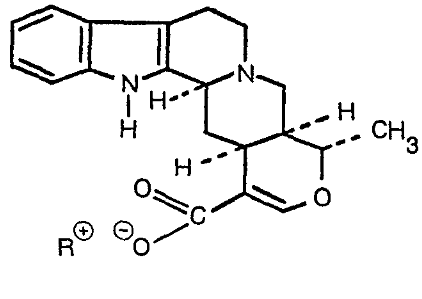

(I)

wherein:

$R^{\oplus}$ represents an alkali or alkaline-earth ion, the ammonium ion or one of the following ions:

a)

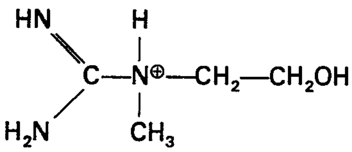

b)

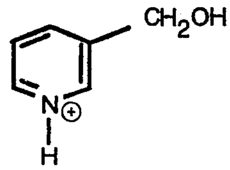

c) $H_3N^{\oplus}—CH_2—CH_2OH$

d)

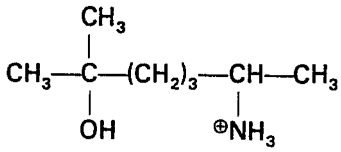

e)

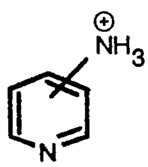

f)

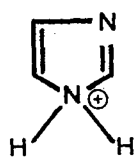

2. A process for the preparation of the compounds corresponding to the general formula I as defined in claim, 1 characterised by a basic hydrolysis of the tetrahydroalstonine corresponding to formula II

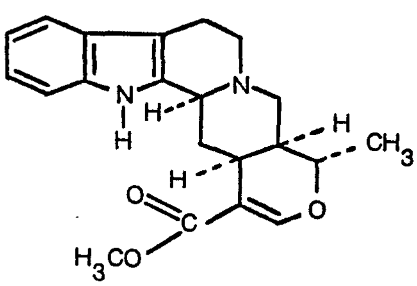

(II)

and, if necessary, by an acidification which produces tetrahydroalstonic acid which is then salified using a suitable mineral or organic base.

3. A process according to claim 2, characterised in that tetrahydroalstonine is saponified by means of soda to obtain sodium tetrahydroalstonate.

4. A process according to claim 3, characterised in that sodium tetrahydroalstonate is trans-salified.

5. A process according to claim 4, characterised in that the trans-salification is carried out using the hydrochloride of the suitable organic base.

6. Compounds corresponding to the general formula I according to claim 1 as new medicaments.

**Claims for the Contracting State: AT**

1. A process for the preparation of compounds corresponding to the general formula I:

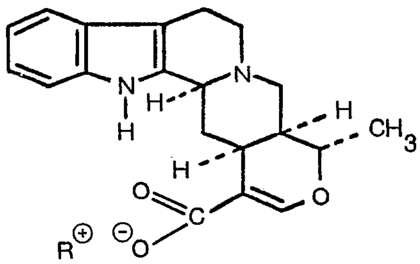

(I)

wherein:

R⊕ represents an alkali or alkaline-earth ion, the ammonium ion or one of the following ions:

a)

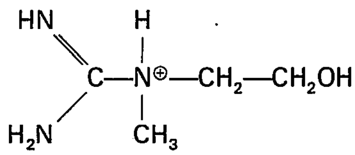

b)

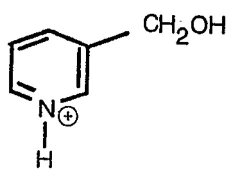

c) $H_3N^{\oplus}—CH_2—CH_2OH$

d)

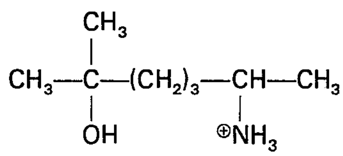

e)

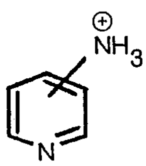

f)

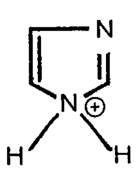

characterised by a basic hydrolysis of the tetrahydroalstonine corresponding to formula II:

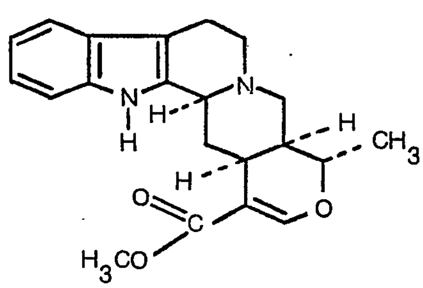

(II)

and, if necessary, by an acidification producing tetrahydroalstonic acid which is then salified using a suitable mineral or organic base.

2. A process according to claim 1, characterised in that tetrahydroalstonine is saponified using soda to obtain sodium tetrahydroalstonate.

3. A process according to claim 2, characterised in that sodium tetrahydroalstonate is trans-salified.

4. A process according to claim 3, characterised in that the trans-salification is carried out using the hydrochloride of the suitable organic base.

**Patentansprüche für die Vertragsstaaten: BE CH DE GB IT LU NL SE**

1. Verbindungen der allgemeinen Formel (I)

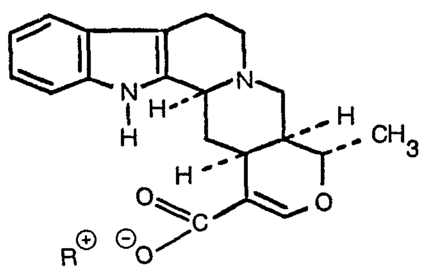

(I)

worin R⊕ ein Alkali- oder Erdalkaliion, das Ammonium ion oder eines der folgenden Ionen darstellt:

a)

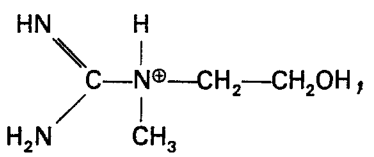

b)

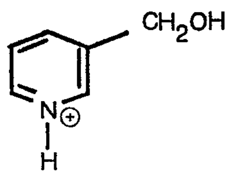

c) $H_3N^{\oplus}—CH_2—CH_2OH$,

d)

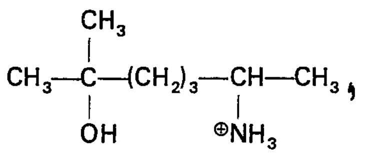

e)

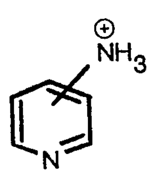

f)

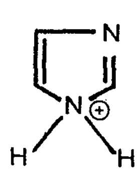

2. Verfahren zur Herstellung von Verbindungen der allgemeinen Formel (I), wie in Anspruch 1 definiert, gekennzeichnet durch eine basische Hydrolyse des Tetrahydroalstonins der Formel (II)

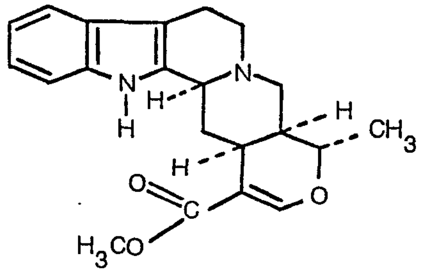

(II)

und gegebenenfalls eine Ansäuerung zur Erzielung der Tetrahydroalstonsäure, welche danach mittels einer geeigneten organischen oder mineralischen Base in ein Salz überführt wird.

3. Verfahren gemäß Anspruch 2, dadurch gekennzeichnet, daß man das Tetrahydroalstonin mittels Soda verseift, um das natriumtetrahydroalstonat zu erhalten.

4. Verfahren gemäß Anspruch 3, dadurch gekennzeichnet, daß das Natriumtetrahydroalstonat in ein anderes Salz überführt wird.

5. Verfahren gemäß Anspruch 4, dadurch gekennzeichnet, daß die Überführung in ein anderes Salz durch das Chlorhydrat der geeigneten organischen Base durchgeführt wird.

6. Die Verbindungen der allgemeinen Formel (I) gemäß Anspruch 1 als neue Heilmittel.

**Patentansprüche für den Vertragsstaat: AT**

1. Verfahren zur Herstellung von Verbindungen der allgemeinen Formel (I)

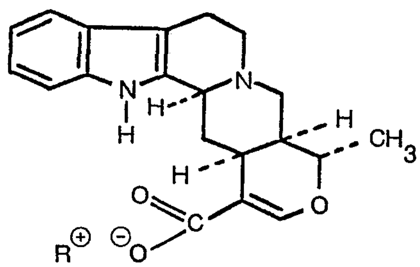

(I)

worin R⊕ ein Alkali- oder Erdalkaliion, das Ammoniumion oder eines der folgenden Ionen darstellt:

a)

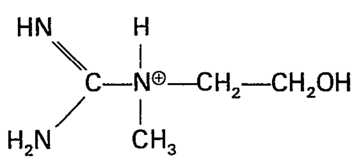

,

b)

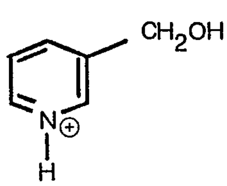

c) $H_3N^{\oplus}—CH_2—CH_2OH$ ,

d)

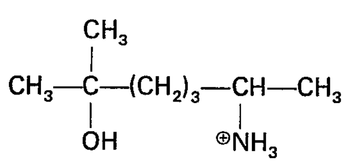

,

e) 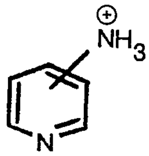

f) 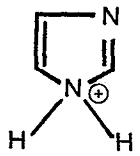

gekennzeichnet durch eine basische Hydrolyse von Tetrahydroalstonin der Formel (II)

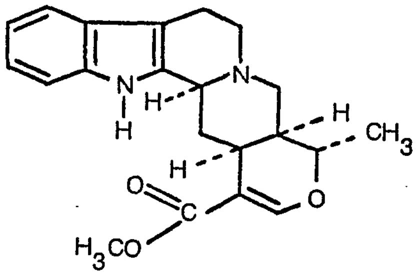

(II)

und gegebenenfalls eine Ansäuerung zur Erzielung der Tetrahydroalstonsäure, welche danach mit einer geeigneten organischen oder mineralischen Base in ein Salz überführt wird.

2. Verfahren gemäß Anspruch 1, dadurch gekennzeichnet, daß man das Tetrahydroalstonin mittels Soda verseift, um das Natriumtetrahydroalstonat zu erhalten.

3. Verfahren gemäß Anspruch 2, dadurch gekennzeichnet, daß das Natriumtetrahydroalstonat in ein anderes Salz überführt wird.

4. Verfahren gemäß Anspruch 3, dadurch gekennzeichnet, daß die Überführung in ein anderes Salz durch das Chlorhydrat der geeigneten organischen Base durchgeführt wird.